(19) Europäisches Patentamt
European Patent Office
Office européen des brevets

(11) Veröffentlichungsnummer : **0 327 981 B1**

# (12) EUROPÄISCHE PATENTSCHRIFT

(45) Veröffentlichungstag der Patentschrift : **06.05.92 Patentblatt 92/19**

(51) Int. Cl.$^5$ : **C07C 255/30,** C07C 255/42, C07C 253/00

(21) Anmeldenummer : **89101853.3**

(22) Anmeldetag : **03.02.89**

(54) **Verfahren zur Herstellung von 2-Cyano-3-aminoacrylnitril-Derivaten.**

(30) Priorität : **12.02.88 DE 3804394**

(43) Veröffentlichungstag der Anmeldung : **16.08.89 Patentblatt 89/33**

(45) Bekanntmachung des Hinweises auf die Patenterteilung : **06.05.92 Patentblatt 92/19**

(84) Benannte Vertragsstaaten : **BE CH DE ES FR GB IT LI NL**

(56) Entgegenhaltungen :
**CH-A- 543 486**
**DE-A- 2 434 922**
**PATENT ABSTRACTS OF JAPAN, unexamined applications, C Field, Band 6, Nr. 139, 28. Juli 1982; THE PATENT OFFICE JAPANESEGO-VERMENT, S. 126 C 117**

(73) Patentinhaber : **BASF Aktiengesellschaft**
**Carl-Bosch-Strasse 38**
**W-6700 Ludwigshafen (DE)**

(72) Erfinder : **Schefczik, Ernst, Dr.**
**Dubliner Strasse 7**
**W-6700 Ludwigshafen (DE)**

EP 0 327 981 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 2-Cyano-3-aminoacrylnitril-Derivaten durch Behandlung von Nitrilen mit einem Alkanol in Gegenwart von Halogenwasserstoff und Umsetzung des resultierenden Iminoester-hydrohalogenids mit Malodinitril in Gegenwart einer Base.

Aus der DE-A-2 434 922 ist die Herstellung von 2-Cyano-3-aminoacrylnitril-Derivaten bekannt. Der dort beschrittene Syntheseweg ist jedoch umständlich, da er über mehrere Stufen führt. Außerdem sind die als Ausgangskomponenten benötigten Carbonsäureanhydride nur in beschränktem Maße zugänglich.

In Synthesis 1981, S. 130ff, wird die Umsetzung von verschiedenen Iminoester-hydrochloriden mit Meldrumsäure (2,3-Dimethyl-1,3-dioxan-4,6-dion) beschrieben. Es wird dort auch erwähnt, daß das Hydrochlorid des Acetimidsäuremethylesters mit Malodinitril unter basischen Bedingungen reagiert. Gleichzeitig wird jedoch darauf hingewiesen, daß man gute Ausbeuten dann erhält, wenn man die Umsetzung mit Meldrumsäure durchführt. Außerdem werden über das Umsetzungsprodukt mit Malodinitril keine Angaben gemacht. Aus. J. Chem. Soc. 1943, S. 388ff, ist in diesem Zusammenhang weiter bekannt, daß die Umsetzung von Formininoether mit Malodinitril zu 4-Amino-5-cyanopyrimidin führt.

Aufgabe der vorliegenden Erfindung war es nun, ein neues Verfahren bereitzustellen, das es erlaubt, 2-Cyano-3-aminoacrylnitril-Derivate auf einem einfachen Syntheseweg herzustellen, wobei die Ausgangsprodukte leicht zugänglich sein sollten.

Es wurde gefunden, daß die Herstellung von 2-Cyano-3-aminoacrylnitril-Derivaten der Formel I

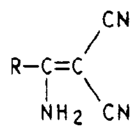 (I),

in der R substituiertes $C_1$-$C_{10}$-Alkyl, gegebenenfalls substituiertes Styryl, gegebenenfalls substituiertes Phenyl oder einen aromatischen heterocyclischen Rest bedeutet, vorteilhaft gelingt, wenn man ein Nitril der Formel II

R-CN (II),

in der R die obengenannte Bedeutung besitzt, in einem inerten organischen Verdünnungsmittel bei einer Temperatur von 0 bis +30°C mit einem gegebenenfalls substituierten $C_1$-$C_6$-Alkanol in Gegenwart von Halogenwasserstoff behandelt und das aus dieser Behandlung resultierende Iminoesterhydrohalogenid der Formel III

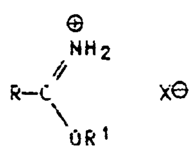 (III),

in der R die obengenannte Bedeutung besitzt und $R^1$ gegebenenfalls substituiertes $C_1$-$C_6$-Alkyl und $X^{\ominus}$ ein Halogenidion bedeuten, gegebenenfalls nach seiner Zwischenisolierung, in einem inerten organischen Verdünnungsmittel bei einer Temperatur von 20 bis 100°C mit Halodinitril in Gegenwart einer Base in neutralem bis alkalischem Milieu umsetzt.

Alle in den obengenannten Formeln auftretenden Alkylreste können sowohl geradkettig als auch verzweigt sein.

Wenn R substituiertes $C_1$-$C_{10}$-Alkyl bedeutet, leiten sich die substituierten $C_1$-$C_{10}$-Alkylreste beispielsweise von folgenden Resten ab: Methyl, Ethyl, Propyl, Isopropyl, Butyl, Isobutyl, sec-Butyl, tert-Butyl, Pentyl, Isopentyl, Neopentyl, tert-Pentyl, Hexyl, Heptyl, Octyl, 2-Ethylhexyl, Nonyl oder Decyl.

Als Substituenten kommen dabei beispielsweise $C_1$-$C_4$-Alkoxy, Phenoxy, Phenyl, das gegebenenfalls durch $C_1$-$C_4$-Alkyl, Nitro, Halogen, insbesondere Fluor, Chlor oder Brom, Hydroxy oder $C_1$-$C_4$-Alkoxy substituiert ist, $C_1$-$C_4$-Hono- oder Dialkylcarbamoyl, Pyrrolidinocarbonyl, Piperidinocarbonyl, Morpholinocarbonyl, Piperazinocarbonyl, N-($C_1$-$C_4$-Alkyl)piperazinocarbonyl, Phenylthio, Phenylsulfonyl, Anilino, $C_1$-$C_4$-Alkanoylamino, Benzaylamino, N-($C_1$-$C_4$-Alkyl)-N-($C_1$-$C_4$-alkanoyl)-amino, N-($C_5$-$C_7$-Cycloalkyl)-N-($C_1$-$C_4$-alkanoyl)amino, N-Phenyl-N-($C_1$-$C_4$-alkanoyl)amino, N-($C_1$-$C_4$-Alkyl)-N-benzoylamina, N-Phenyl-N-benzoylamino, Phenylsulfonylamino, N-($C_1$-$C_4$-Alkyl)-N-phenylsulfonylamino, N-Phenyl-N-phenylsulfonylamino, N,N′-($C_1$-$C_4$-Dialkyl)ureido, N-Phenyl-N′-($C_1$-$C_4$-alkyl)ureido, N,N′-Diphenylureido, 4,5-Dichlorpyridazin-1-yl oder ein Rest der Formel

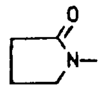 , 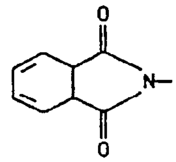 , 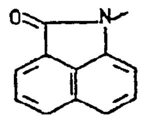 oder 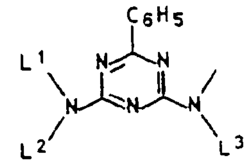 ,

wobei $L^1$, $L^2$ und $L^3$ gleich oder verschieden sind und unabhängig voneinander jeweils für Wasserstoff oder $C_1$-$C_4$-Alkyl stehen, in Betracht.

R bedeutet weiterhin z.B. unsubstituiertes Phenyl, durch $C_1$-$C_4$-Alkyl, $C_1$-$C_4$-Alkoxy, Halogen, insbesondere Fluor, Chlor oder Brom, Hydroxy, Phenoxy, Phenylthio, Phenylsulfonyl, $C_1$-$C_4$-Alkoxycarbonyl, $C_1$-$C_4$-Hydroxyalkyl, $C_1$-$C_4$-Halogenalkyl, $C_1$-$C_4$-(Pyrrolidino)-, -(Piperidino)-, -(Morpholino)-, -(Piperazino)- oder -[N-($C_1$-$C_4$-Alkyl)piperazino]alkyl, $C_1$-$C_4$-Phenylalkoxy, $C_1$-$C_4$-Dialkylsulfamoyl oder $C_1$-$C_4$-Alkanoylamino substituiertes Phenyl, Styryl, durch $C_1$-$C_4$-Dialkylamino substituiertes Styryl, Pyridinyl, durch $C_1$-$C_4$-Alkyl substituiertes Pyridinyl, Thienyl oder durch $C_1$-$C_4$-Alkyl substituiertes Thienyl.

Eine bevorzugte Form des erfindungsgemäßen Verfahrens besteht darin, daß man Nitrile der Formel II verwendet, in der R 3-($C_1$-$C_4$-Alkoxy)propyl, Benzyl oder Phenyl bedeutet.

Geeignete $C_1$-$C_6$-Alkanole sind z.B. Methanol, Ethanol, Propanol, Isopropanol, Butanol, Isobutanol, sec-Butanol, Pentanol, Isopentanol, Neopentanol oder Hexanol. Als gegebenenfalls substituiertes $C_1$-$C_6$-Alkanol ist z.B 2-Chlorethanol zu nennen. Die Verwendung von Methanol, Ethanol oder Propanol ist bevorzugt.

Als Halogenwasserstoff eignet sich z.B. Fluorwasserstoff, Chlorwasserstoff oder Bromwasserstoff. Die Verwendung von Chlorwasserstoff ist bevorzugt.

Inerte organische Verdünnungsmittel, die im erfindungsgemäßen Verfahren zur Anwendung kommen können, sind z.B. Kohlenwasserstoffe, die gegebenenfalls halogeniert, insbesondere chloriert sind, wie Toluol, Xylol, Chloroform, 1,1,1-Trichlorethan, 1,2-Dichlorethen, 1,2-Dichlorethan, Chlorbenzol oder 1,2-Dichlorbenzol, oder Ether, wie Diethylether, Methyl-tert-butylether, Tetrahydrofuran oder Dioxan.

Es ist auch möglich Alkanole, z.B. Methanol oder Ethanol, als Verdünnungsmittel zu verwenden. In diesem Fall dient der Alkohol gleichzeitig als Reaktionspartner und als Verdünnungsmittel.

Geeignete Basen für das neue Verfahren sind z.B. anorganische Basen, wie Natrium- oder Kaliumcarbonat oder Natrium- oder Kaliumhydrogencarbonat oder organische Basen, beispielsweise tertiäre Amine, wie Triethylamin, Tripropylamin, Triisopropylamin, Tributylamin, N,N-Dimethylanilin oder N,N-Diethylanilin oder Alkalialkanolate, wie Natriummethanolat oder Natriumethanolat.

Das erfindungsgemäße Verfahren wird zweckmäßig so durchgeführt, daß man Nitril II und $C_1$-$C_6$-Alkanol im Molverhältnis 1:1 bis 1:5, vorzugsweise 1:1 bis 1:2, zusammen mit den inerten Verdünnungsmittel vorlegt und in diese Mischung unter unter Rühren 1 bis 5 Mol, vorzugsweise 1 bis 2 Mol, Halogenwasserstoff bei einer Temperatur von 0 bis +30°C einleitet. Nach einer Reaktionsdauer von ca. 2 bis 20 Stunden wird das resultierende Iminoester-hydrohalogenid III, gegebenenfalls nach seiner Zwischenisolierung, mit Malodinitril umgesetzt.

Das Iminoester-hydrohalogenid III liegt, abhängig von der Art des inerten Verdünnungsmittels, entweder in festem Aggregatzustand oder als Lösung vor. In manchen Fällen kann es von Vorteil sein, das Iminoester-hydrohalogenid III zwischenzuisolieren. Bei einer solchen Zwischenisolierung wird es entweder abgesaugt oder man entfernt das Verdünnungsmittel unter vermindertem Druck.

Eine bevorzugte Verfahrensweise besteht darin, das Iminoester-hydrohalogenid III ohne Zwischenisolierung direkt weiter umzusetzen. Dabei wird das Reaktionsgemisch entweder zuerst mit Malodinitril und anschließend mit der Base oder vorzugsweise zuerst mit der Base und anschließend mit Malonitril versetzt.

Je Mol Nitril II verwendet man dabei 1 bis 2 Mol, vorzugsweise 1 bis 1,1 Mol Malodinitril und 1 bis 2 Mol, vorzugsweise 1 bis 1,2 Mol, Base.

Wenn man das Iminoester-hydrohalogenid III zwischenisoliert hat, wird es zunächst wieder mit einem inerten organischen Verdünnungsmittel und anschließend, wie oben beschrieben, mit Malodinitril und Base versetzt.

Das Reaktionsgemisch wird dann unter Rühren auf eine Temperatur von 20 bis 100°C, vorzugsweise 20 bis 80°C erhitzt und ca. 1 bis 10 Stunden bei dieser Temperatur belassen. Wie oben bereits ausgeführt, ist dabei zu beachten, daß dieser Reaktionsschritt in neutralem bis alkalischem Milieu abzulaufen hat.

Wenn das inerte Verdünnungsmittel mit Wasser mischbar ist, wird das Reaktionsgemisch nach dem Abkühlen mit Wasser verdünnt, wobei das Zielprodukt I ausfällt. Für den Fall, daß es sich um ein nicht mit Wasser mischbares Verdünnungsmittel handelt, empfiehlt es sich, das Verdünnungsmittel mittels Wasserdampfdestillation zu entfernen.

Das so erhaltene 2-Cyano-3-aminoacrylnitril-Drivat kann nach dem Trocknen entweder direkt für weitere

Synthesen verwendet oder auf an sich bekannte Weise weiter gereinigt werden.

Die Vorteile des neuen Verfahrens, das sowohl in kontinuierlicher als auch diskontinuierlicher Arbeitsweise durchgeführt werden kann, liegen darin, daß die Zielprodukte der Formel I auf einfache Weise und in guter Ausbeute und hoher Reinheit erhalten werden. Zudem ist das neue Verfahren in breitem Bereich anwendbar, da die als Ausgangsprodukte dienenden Nitrile der Formel II im allgemeinen gut zugänglich sind.

Die mittels des erfindungsgemäßen Verfahrens hergestellten 2-Cyano-3-aminoacrylnitril-Derivate der Formel I sind wertvolle Zwischenprodukte für die Synthese von Farbstoffen, Pharmazeutika oder Pflanzenschutzmittel.

Beispielsweise können sie auf an sich bekannte Weise, wie in der DE-A-2 434 922 beschrieben, mit Schwefelwasserstoff in die entsprechenden 2-Cyano-3-aminoacrylsäurethioamid-Derivate der Formel IV

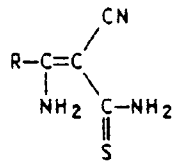

in der R die obengenannte Bedeutung besitzt, übergeführt werden, die wiederum nach bekannten Verfahren, wie in der DE-A-2 434 922 beschrieben, durch oxidativen Ringschluß, beispielsweise mit wäßriger Wasserstoffperoxidlösung, in Isothiazole der Formel V

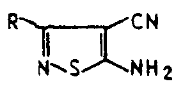 (V),

in der R die obengenannte Bedeutung besitzt, umgewandelt werden können. Die Isothiazole V sind wertvolle Diazokomponenten für Azofarbstoffe (siehe z.B. Dyes and Pigments 3, 106 (1982)).

Die folgenden Beispiele sollen die Erfindung näher erläutern.

Beispiel 1

In eine Lösung von 206 g Benzonitril und 128 g Methanol in 500 ml Toluol wurden bei 5 bis 10°C 140 g gasförmiger Chlorwasserstoff eingeleitet. Man rührte 16 Stunden bei Raumtemperatur nach und entfernte dann überschüssigen Chlorwasserstoff während 4 Stunden unter vermindertem Druck. Dann gab man 300 g Methanol zu und ließ unter Kühlen bei 10 bis 20°C 240 g Triethylamin bis zu einem pH-Wert von 7,5 bis 8 zulaufen. Nach Zugabe von 132 g Malodinitril wurde 2 Stunden unter Rückfluß gekocht, dann mit 400 ml Wasser versetzt und das Toluol mit Wasserdampf abdestilliert. Nach dem Erkalten wurde das auskristallisierte Produkt abgesaugt und mit Wasser gewaschen. Nach dem Trocknen erhielt man 315 g 2-Cyano-3-amino-3-phenyl-acrylnitril, vom Schmelzpunkt 182 bis 183°C (aus Ethanol).

Analyse:

$C_{10}H_7N_3$ (169)

| | | | |
|---|---|---|---|
| ber.: | C 71.0 | H 4.2 | N 24.8 |
| gef.: | 71.1 | 4.3 | 25.0 |

Beispiel 2

Beispiel 2 wurde analog Beispiel 1 durchgeführt, jedoch wurde das Iminoester-hydrochlorid durch Absaugen zwischenisoliert.

132 g Malodinitril wurden dann in 500 ml Methanol gelöst. In die Lösung wurden unter Rühren bei 10 bis 20°C zuerst 343 g Benzoeimidsäuremethylester-hydrochlorid eingetragen und dann 205 g Triethylamin zugetropft. Nun kochte man 4 Stunden unter Rückfluß und destillierte das Methanol mit Wasserdampf ab. Nach dem Erkalten wurde die Kristallsuspension abgesaugt, die Kristalle mit Wasser gewaschen und getrocknet. Man erhielt 337 g 2-Cyano-3-amino-3-phenylacrylnitril (identisch mit der in Beispiel 1 erhaltenen Verbindung).

Beispiel 3

103 g Benzonitril wurden mit 64 g Methanol vermischt und bei 10 bis 15°C solange mit gasförmigem Chlorwasserstoff begast, bis die Gewichtszunahme 70 g betrug. Man ließ unter Rühren 16 Stunden bei Raumtemperatur nachreagieren und zog dann überschüssigen Chlorwasserstoff und Methanol unter vermindertem Druck ab. Der verbleibende Salzbrei wurde mit 300 ml Methanol in Lösung gebracht und durch Zutropfen von 190 g 30 gew.%iger methanolischer Natriummethanolatlösung bei 10 bis 20°C alkalisch gestellt. Dann gab man 66 g Malodinitril zu und rührte 4 Stunden bei 50 bis 60°C. Während des Erkaltens verdünnte man mit 1000 ml Wasser und stellt das Reaktionsgemisch durch Zugabe von Salzsäure neutral. Der gebildete Niederschlag wurde abgesaugt, mit Wasser gewaschen und getrocknet. Man erhielt 151 g 2-Cyano-3-amino-3-phenylacrylnitril mit den im Beispiel 1 angegebenen Eigenschaften.

(Anstelle von Natriummethanolat lassen sich auch Natriumcarbonat oder Kaliumcarbonat verwenden.)

Beispiel 4

In ein Gemisch aus 880 ml Chloroform, 234 g Benzylcyanid und 65 g Methanol wurden bei 5 bis 10°C 90 g gasförmiger Chlorwasserstoff eingeleitet. Danach ließ man bei 10°C 12 Stunden stehen und blies zur Entfernung des überschüssigen Chlorwasserstoffs anschließend mehrere Stunden trockenen Stickstoff durch das Reaktionsgemisch. Dann wurden 280 g Triethylamin bei 10 bis 20°C zugetropft. Nach Zugabe von 132 g Malodinitril wurde 6 Stunden unter Rückfluß gekocht und dann das Chloroform mit Wasserdampf abdestilliert. Es resultiere eine wäßrige Lösung, die beim Erkalten Kristalle abschied. Man verdünnte mit dem gleichen Volumen an kaltem Wasser, saugte ab und wusch mit Wasser. Nach dem Trocknen erhielt man 353 g 2-Cyano-3-amino-3-benzylacrylnitril in Form farbloser Kristalle. Eine aus Ethanol umkristallisierte Probe schmilzt bei 162 bis 163°C und zeigt folgende

Analyse:

$C_{11}H_9N_3$ (183)

| | | | |
|---|---|---|---|
| ber.: | C 72.1 | H 5.0 | 22.9 |
| gef.: | 72.0 | 5.0 | 22.8 |

Beispiel 5

In ein bei 10 bis 15°C gerührtes Gemisch aus 100 ml Dioxan, 32 g Methanol und 151,5 g 2-Chlorbenzylcyanid wurde so lange gasförmiger Chlorwasserstoff eingeleitet, bis die Gewichtszunahme 50 g betrug. Man rührte dann 16 Stunden bei Raumtemperatur nach und entfernte überschüssigen Chlorwasserstoff unter vermindertem Druck. Zu dem resultierenden Kristallbrei goß man eine Lösung von 66 g Malodinitril in 200 ml Methanol, kühlte und tropfte anschließend bei 15 bis 20°C 125 g Triethylamin zu. Dann erhitze man 4 Stunden zum Sieden und trug das Reaktionsgemisch auf 1000 ml Wasser aus. Nach dem Absaugen und Trocknen erhielt man 214 g der Verbindung der Formel

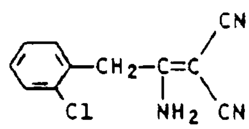

mit einem Schmelzpunkt von 162 bis 163 (aus Ethanol).

(Cl ber 16,3 gef. 16,3)

Die folgende Tabelle enthält weitere 2-Cyano-3-aminoacrylnitrile der Formel

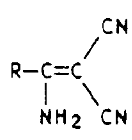

die in analoger Weise erhalten wurden.

| Beispiel | R | hergestellt nach Bsp. | Fp [°C] (Lösungsmittel, aus dem umkristallisiert wurde) |
|---|---|---|---|
| 6 | 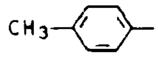 | 1 | 150-151 (Pentanol) |
| 7 | 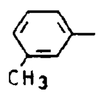 | 2 | 188-189 (Pentanol) |
| 8 | 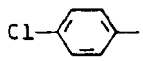 | 1 | 224-225 (Chlorbenzol) |
| 9 | 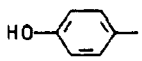 | 1 | 268-269 (Pentanol) |
| 10 | 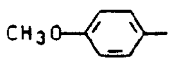 | 2 | 193-194 (Chlorbenzol) |
| 11 | 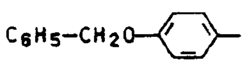 | 1 | 173-174 (Ethanol) |
| 12 | 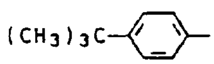 | 3 | 154-155 (Xylol) |
| 13 | 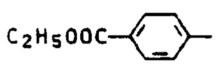 | 1 | 170-171 (Ethanol) |
| 14 | 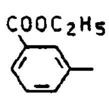 | 1 | 163-164 (Ethanol) |
| 15 | 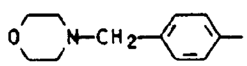 | 1 | 184-185 (Propanol) |
| 16 | 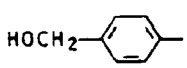 | 1 | 241-242 (Essigsäure) |
| 17 | 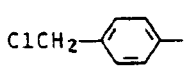 | 1 | 143-144 (Ethanol) |
| 18 | 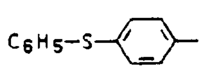 | 1 | 195-196 (Chlorbenzol) |
| 19 | 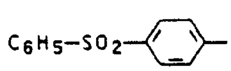 | 1 | 243-244 (Essigsäure) |

| Beispiel | R | hergestellt nach Bsp. | Fp [°C] (Lösungsmittel, aus dem umkristallisiert wurde) |
|---|---|---|---|
| 20 | 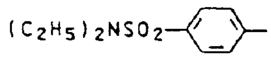 | 1 | 195-196 (Butanol) |
| 21 | 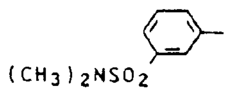 | 1 | 219-220 (Pentanol) |
| 22 | 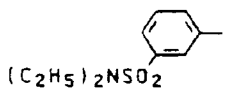 | 1 | 166-167 (Ethanol) |
| 23 | 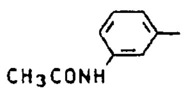 | 1 | 243-244 (Propanol) |
| 24 | 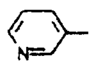 | 1 | 216-217 (Ethanol) |
| 25 | 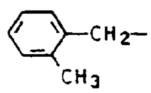 | 4 | 164-165 (Methanol) |
| 26 | 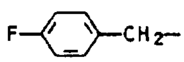 | 1 | 163-164 (Ethanol) |
| 27 | 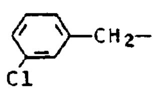 | 4 | 164-165 (Ethanol) |
| 28 | 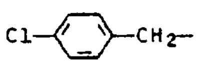 | 1 | 165-166 (Ethanol) |
| 29 | 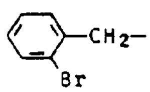 | 1 | 153-154 (Ethanol) |
| 30 | 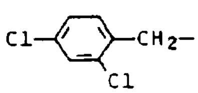 | 1 | 180-181 (Pentanol) |
| 31 | 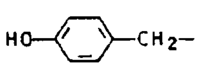 | 1 | 207-208 (Pentanol) |
| 32 | 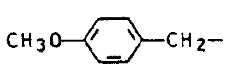 | 5 | 154-155 (Ethanol) |

| Beispiel | R | hergestellt nach Bsp. | Fp [°C] (Lösungsmittel, aus dem umkristallisiert wurde) |
|---|---|---|---|
| 33 | 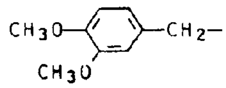 | 4 | 163-164 (Essigsäure) |
| 34 | 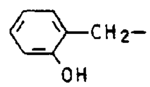 | 1 | 161-162 (Ethanol) |
| 35 | 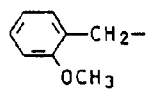 | 5 | 117-118 (Toluol) |
| 36 | 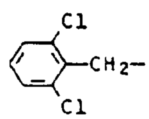 | 1 | 227-228 (Essigsäure) |
| 37 | 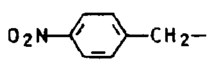 | 1 | 187-188 (Pentanol) |
| 25 | 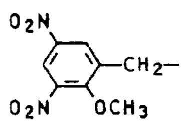 | 1 | 161-162 (Ethanol) |
| 39 | $CH_3OCH_2CH_2CH_2-$ | 4 | 93- 94 (Toluol) |
| 40 | 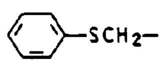 | 1 | 131-132 (Methanol) |
| 41 | 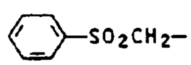 | 1 | 248-249 (Pentanol) |
| 42 | 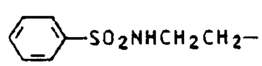 | 1 | 134-135 (Ethanol) |
| 43 | 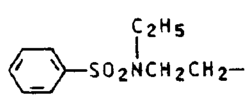 | 1 | 150-151 (Methanol) |
| 44 | 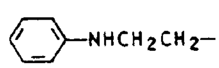 | 1 | 261-262 (γ-Butyrolacton) |
| 45 | $C_4H_9NHCOCH_2-$ | 1 | 129-130 (Methanol) |
| 46 | $(CH_3)_2NCOCH_2-$ | 1 | 172-173 (Pentanol) |

| Beispiel | R | hergestellt nach Bsp. | Fp [°C] (Lösungsmittel, aus dem umkristallisiert wurde) |
|---|---|---|---|
| 47 | 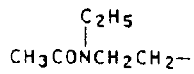 | 4 | 179-180 (Ethanol) |
| 48 | 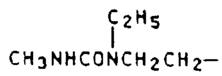 | 1 | 178-179 (Essigsäure) |
| 49 | 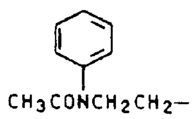 | 1 | 202-203 (Pentanol) |
| 50 | 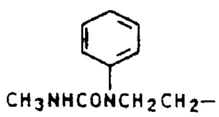 | 1 | 226-227 (Essigsäure) |
| 51 | 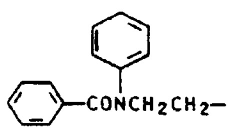 | 1 | 225-226 (Pentanol) |
| 52 | 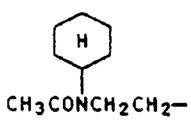 | 5 | 192-193 (Propanol) |
| 53 | 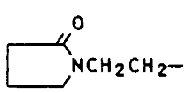 | 4 | 243-244 (Ethanol) |
| 54 | 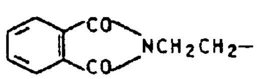 | 1 | 274 (Zers.) (N,N-Dimethylformamid) |
| 55 | 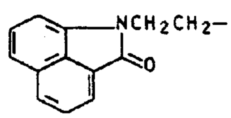 | 1 | 285-286 (Essigsäure) |
| 56 | 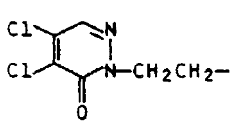 | 1 | 233-234 (Pentanol) |

Anwendungsbeispiel

a) 169 g 2-Cyano-3-amino-3-phenylacrylnitril wurden in 300 ml N-Methylpyrrolidon gelöst und nach Zugabe von 30 g Triethylamin bei 80°C mit Schwefelwasserstoff begast. Bei einer Gewichtszunahme von 34 g stoppte man die Gasaufnahme. Man rührte das Reaktionsgemisch in 1000 ml Eiswasser und 50 ml konz.

Salzsäure ein und saugte das ausgefallene Produkt ab. Nach dem Waschen mit Wasser und Trocknen erhielt man 180 g 2-Cyano-3-amino-3-phenylacrylsäurethioamid der Formel

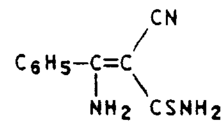

Ein aus Ethanol umkristallisierte Probe schmilzt bei 217-218°C und zeigt folgende Analysenwerte

$C_{10}H_9N_3S$ (293)

| | | | | |
|---|---|---|---|---|
| ber.: | C 59.1 | H 4.5 | N 20.7 | S 15.8 |
| gef.: | 59.1 | 4.4 | 20.8 | 15.8 |

b) 203 g 2-Cyano-3-amino-3-phenylacrylsäurethioamid wurden in 1000 ml Ethanol zum Sieden erhitzt. Dazu wurden 150 g 30 gew.%iges wäßriges Wasserstoffperoxid in dem Maße zugetropft, daß das Reaktionsgemisch ohne äußere Heizung am Sieden bleibt (1 Stunde). Danach wurde noch 2 Stunden am Sieden gehalten und anschließend unter Rühren erkalten gelassen. Die Kristallisation wurde durch Zugabe von 2000 ml Wasser vervollständigt. Nach dem Absaugen, Waschen und Trocknen erhielt man 189 g 3-Phenyl-4-cyano-5-aminoisothiazol vom Schmelzpunkt 179-180°C (aus Ethanol). Analyse:

$C_{10}H_7N_3S$ (201)

| | | | | |
|---|---|---|---|---|
| ber.: | C 59.7 | H 3.5 | N 20.9 | S 15.9 |
| gef.: | 59.9 | 3.5 | 20.7 | 15.6 |

## Patentansprüche

1. Verfahren zur Herstellung von 2-Cyano-3-aminoacrylnitril-Derivaten der Formel I

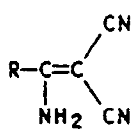 (I),

in der R substituiertes $C_1$-$C_{10}$-Alkyl, gegebenenfalls substituiertes Styryl, gegebenenfalls substituiertes Phenyl oder einen aromatischen heterocyclischen Rest bedeutet, dadurch gekennzeichnet, daß man ein Nitril der Formel II

R-CN (II),

in der R die obengenannte Bedeutung besitzt, in einem inerten organischen Verdünnungsmittel bei einer Temperatur von 0 bis +30°C mit einem gegebenenfalls substituierten $C_1$-$C_6$-Alkanol in Gegenwart von Halogenwasserstoff behandelt und das aus dieser Behandlung resultierende Iminoester-hydrohalogenid der Formel III

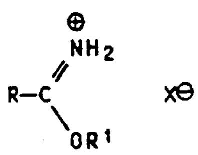 (III),

in der R die obengenannte Bedeutung besitzt und $R^1$ gegebenenfalls substituiertes $C_1$-$C_6$-Alkyl und $X^{\ominus}$ ein Halogenidion bedeuten, gegebenenfalls nach seiner Zwischenisolierung, in einem inerten organischen Verdünnungsmittel bei einer Temperatur von 20 bis 100°C mit Halodinitril in Gegenwart einer Base in neutralem bis alkalischem Milieu umsetzt.

2. Verfahren gemäß Anspruch 1, dadurch gekennzeichnet, daß man ein Nitril der Formel II verwendet, in der R 3-($C_1$-$C_4$-Alkoxy)propyl, Benzyl oder Phenyl bedeutet.

3. Verfahren gemäß Anspruch 1, dadurch gekennzeichnet, daß man das Nitril der Formel II mit Methanol, Ethanol oder Propanol und Chlorwasserstoff umsetzt.

4. Verfahren gemäß Anspruch 1, dadurch gekennzeichnet, daß man das Iminoester-hydrohalogenid der

Formel III ohne Zwischenisolierung weiter umsetzt.

## Claims

1. A process for preparing a 2-cyano-3-aminoacrylonitrile derivative of the formula I

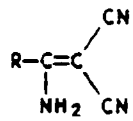

(I)

where R is substituted $C_1$-$C_{10}$-alkyl, substituted or unsubstituted styryl, substituted or unsubstituted phenyl or aromatic heterocyclyl, which comprises treating a nitrile of the formula II

R-CN (II)

where R is as defined above, in an inert organic diluent at from 0 to +30°C with a substituted or unsubstituted $C_1$-$C_6$-alkanol in the presence of a hydrogen halide and reacting the resulting iminoester hydrohalide of the formula III

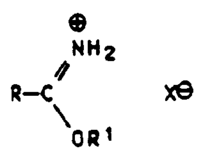

(III)

where R is as defined above, $R^1$ is substituted or unsubstituted $C_1$-$C_6$-alkyL and $X^{\ominus}$ is a halide ion, with or without intermediary isolation, with malodinitrile in a neutral or alkaline medium in an inert organic diluent at from 20 to 100°C in the presence of a base.

2. A process as claimed in claim 1, wherein the nitrile used is of the formula II where R is 3-($C_1$-$C_4$-alkoxy)propyl, benzyl or phenyl.

3. A process as claimed in claim 1, wherein the nitrile of the formula II is reacted with methanol, ethanol or propanol and hydrogen chloride.

4. A process as claimed in claim 1, wherein the iminoester hydrohalide of the formula III is further reacted without being first isolated.

## Revendications

1. Procédé de polymérisation de dérivés du 2-cyano-3-aminoacrylonitrile de la formule I:

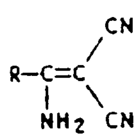

(I),

dans laquelle R représente un radical alkyle en $C_1$-$C_{10}$ substitué, styryle éventuellement substitué, phényle éventuellement substitué ou un radical aromatigue hétérocyclique, caractérisé en que l'on traite un nitrile de la formule II:

R-CN (II)

dans laquelle R possède les significations lui attribuées ci-dessus, dans un solvant inerte et à une température de 0 à +30°C, par un alcanol en $C_1$-$C_6$ éventuellement substitué, en présence d'un hydrocarbure halogéné et on fait réagir l'halogénhydrate d'iminoester obtenu par ce traitement, répondant à la formule III:

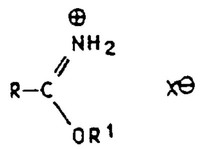

(III),

dans laquelle R possède les significations lui attribuées ci-dessus et $R_1$ représente un radical allyle en $C_1$-$C_6$ éventuellement substitué et $X^{\ominus}$ représente un ion halogénure, éventuellement après son isolement intermédiaire, dans un diluant organique inerte, à une température de 20 à 100°C, avec le malodinitrile, en présence d'une base, en milieu alcalin à neutre.

2. Procédé suivant la revendication 1, caractérisé en ce que l'on utilise un nitrile de la formule II dans laquelle R représente un radical 3-(alcoxy en $C_1$-$C_4$)-propyle, benzyle ou phényle.

3. Procédé suivant la revendication 1, caractérisé en que l'on fait réagir le nitrile de la formule II avec le méthanol, l'éthanol ou le propanol et l'acide chlorhydrique.

4. Procédé suivant la revendicaiion 1, caractérisé en ce que l'on fait réagir l'halogénhydrate d'iminoester de la formule III sans procéder à son isolement intermédiaire.